(19) Europäisches Patentamt
European Patent Office
Office européen des brevets

(11) EP 3 119 378 B1

(12) # EUROPEAN PATENT SPECIFICATION

(45) Date of publication and mention of the grant of the patent:
**25.04.2018 Bulletin 2018/17**

(21) Application number: **15713113.7**

(22) Date of filing: **16.03.2015**

(51) Int Cl.:
***A61K 8/41*** *(2006.01)* ***A61Q 5/00*** *(2006.01)*
***A61K 8/92*** *(2006.01)*

(86) International application number:
**PCT/US2015/020643**

(87) International publication number:
**WO 2015/142687 (24.09.2015 Gazette 2015/38)**

(54) **HAIR OIL CONDITIONING AGENT**

HAARÖLKONDITIONIERUNGSMITTEL

AGENT DE CONDITIONNEMENT HUILEUX POUR LES CHEVEUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.03.2014 IN 830DE2014**

(43) Date of publication of application:
**25.01.2017 Bulletin 2017/04**

(73) Proprietor: **Dow Global Technologies LLC**
**Midland, MI 48674 (US)**

(72) Inventors:
- **GADGIL, Sandeep V.**
  **Thane 400602 (IN)**
- **NAIR, Rejith R.**
  **Thane 400601 (IN)**
- **PILLAY, Shamit**
  **Maharashtra 400101 (IN)**
- **SAWANT, Mahesh R.**
  **Mumbai 400042 (IN)**

(74) Representative: **Houghton, Mark Phillip et al**
**Patent Outsourcing Limited**
**1 King Street**
**Bakewell, Derbyshire DE45 1DZ (GB)**

(56) References cited:
**WO-A2-2014/027120**
**JP-A- 2008 137 916**
**JP-B1- S4 619 639**
**US-A1- 2006 088 495**
**US-A1- 2012 009 127**

- **DATABASE GNPD [Online] MINTEL; 1 February 2014 (2014-02-01), "Conditioner", XP002740350, Database accession no. 2308907**
- **DATABASE GNPD [Online] MINTEL; 1 March 2013 (2013-03-01), "Hands & Nails Cream", XP002740351, Database accession no. 2027595**
- **Anonymous: "Dihydroxypropyltrimonium Chloride - "Glycerol Quat"", CosmetoScope , 4 May 2011 (2011-05-04), XP055189249, Retrieved from the Internet: URL:http://www.cosmetoscope.com/2010/03/dihydroxypropyltrimonium-chloride-glycerol-quat/ [retrieved on 2015-05-13]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

This invention relates to a hair oil formulation with a conditioning agent and a method for preparing a conditioned hair oil.

Hair oil treatments provide nourishment and conditioning to hair, but leave a residual greasy feel and are difficult to remove without depleting moisture in the hair. Glycerol Quat (2,3-dihydroxypropyl trimethylammonium chloride) has been used as a moisturizing agent in skin creams, as disclosed in US7282471. However, the prior art does not address the problem of improving hair oil formulations.

The problem addressed by the present invention is to provide an improved hair oil formulation.

## STATEMENT OF INVENTION

The present invention provides a composition comprising: (a) from 60 to 85 wt% vegetable oil; (b) an aliphatic quaternary ammonium salt having from five to twenty carbon atoms and from two to six hydroxyl groups; and (c) a surfactant package.

The present invention is further directed to a method for preparing a conditioned hair oil. The method comprises combining with a hair oil: (a) an aliphatic quaternary ammonium salt having from five to twenty carbon atoms and from two to six hydroxyl groups; and (b) a surfactant package.

## DETAILED DESCRIPTION

Percentages are weight percentages (wt%), temperatures are in °C and results were obtained at room temperature (20-25°C), unless specified otherwise. Percentages listed with formulated ingredients are the percent solids of the aqueous solution or dispersion. Percentages in the composition are based on the entire composition including water and oil. HLB value (hydrophile-lipophile balance) is a measure of the degree to which a nonionic surfactant is hydrophilic or lipophilic. It is expressed as percentage by weight of the hydrophilic portion of the surfactant molecule:

$$HLB = \frac{100}{5} \times \frac{molecular\ weight\ of\ hydrophilic\ portion}{total\ molecular\ weight}$$

The HLB values of all nonionic surfactants are typically verified by experimental techniques by the manufacturer. The HLB numbers used in the example are from the data reported by ICI Americas Inc (Ref: "The HLB SYSTEM a time-saving guide to emulsifier selection, ICI Americas Inc. Wilmington, Delaware) For purposes of this invention, aliphatic compounds are compounds containing no aromatic rings, i.e., they may include both straight or branched chains and non-aromatic rings. Alkyl groups are straight or branched chain hydrocarbon groups.

The composition of this invention may contain more than one vegetable oil. Preferred vegetable oils may include, e.g., coconut oil, sesame oil, mustard oil, argan oil, olive oil, almond oil, safflower oil and avocado oil; preferably coconut oil. The vegetable oil is present in the composition in an amount from 60 to 85 wt%, preferably at least 65 wt%, preferably at least 67 wt%, preferably at least 69 wt%; preferably no more than 80 wt%, preferably no more than 78 wt%, preferably no more than 76 wt%, preferably no more than 74 wt%.

Preferably, the aliphatic quaternary ammonium salt has no more than eighteen carbon atoms, preferably no more than sixteen, preferably no more than fourteen, preferably no more than twelve, preferably no more than ten, preferably no more than nine, preferably no more than eight; preferably at least six. Preferably, the aliphatic quaternary ammonium salt has no more than five hydroxyl groups, preferably no more than four, preferably no more than three. Preferably, the ratio of the number of carbon atoms in the salt to the number of hydroxyl groups is from 1.5:1 to 6:1, preferably from 1.7:1 to 4.5:1, preferably from 2:1 to 4:1, preferably from 2.5:1 to 3.5:1. Preferably, no carbon atom in the aliphatic quaternary ammonium salt is attached to more than one hydroxyl group. Preferably, at least one methyl group is attached to the quaternary nitrogen atom, preferably at least two, preferably three. Preferably, the aliphatic quaternary ammonium salt has only one nitrogen atom. Preferably, the aliphatic quaternary ammonium salt is an acyclic compound. Preferably, the aliphatic quaternary ammonium salt is a saturated aliphatic compound. Preferably, the aliphatic quaternary ammonium salt has no non-hydrocarbon substituents other than the quaternary nitrogen and the hydroxyl groups. Preferably, the aliphatic quaternary ammonium salt has at least one 2,3-dihydroxypropyl group attached to the quaternary nitrogen atom. The anion of the aliphatic quaternary ammonium salt preferably is halide, preferably chloride. Aliphatic quaternary ammonium salts and their methods of preparation are disclosed, e.g., in WO2012050769.

Preferred aliphatic quaternary ammonium salts include, e.g.,

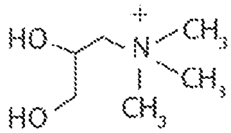

2,3-dihydroxylpropyl trimethylammonium chloride (Glycerol Quat)

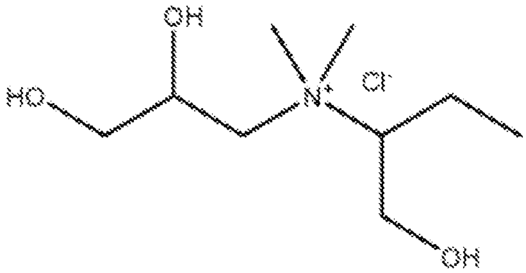

2-(2,3-dihydroxypropyldimethylammonmm)-1-butanol chloride

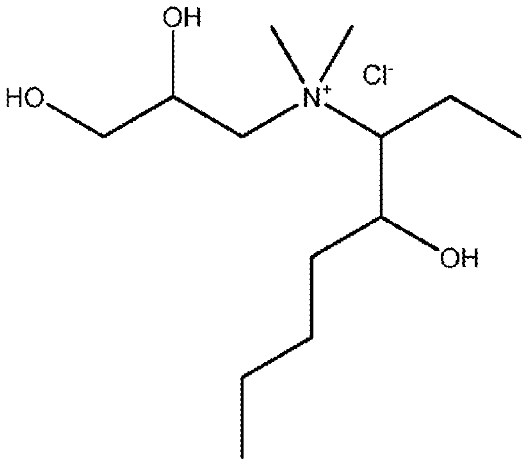

3-(2,3 dihydroxypropyldimethylammonium)-4-hydroxyoctane chloride

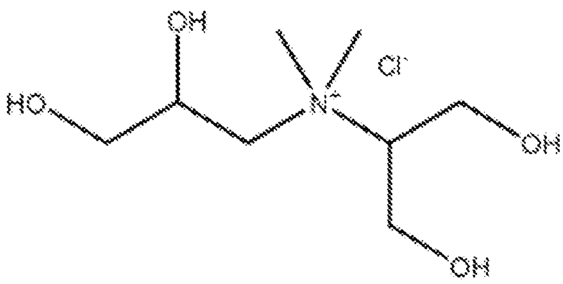

2-(2,3 dihydroxypropyldimethylammonium)-1,3-propanediol chloride

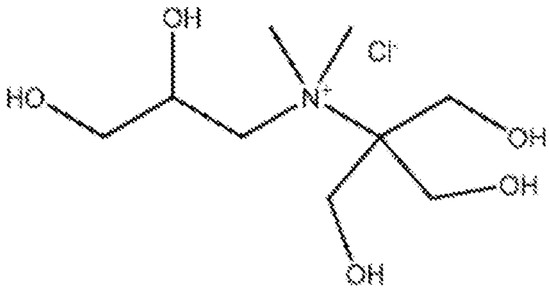

2-(2,3-dihydroxypropyldimethylammonium)-2-hydroxymethyl-1,3-propanediol chloride

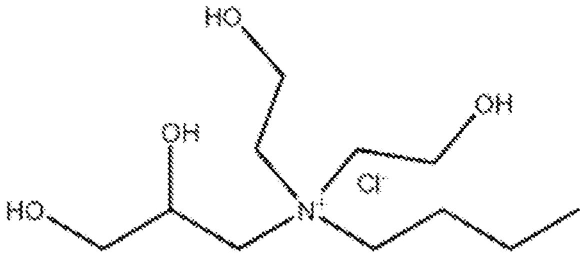

2,3 dihydroxypropyl(di)hydroxyethylbutylammonium chloride

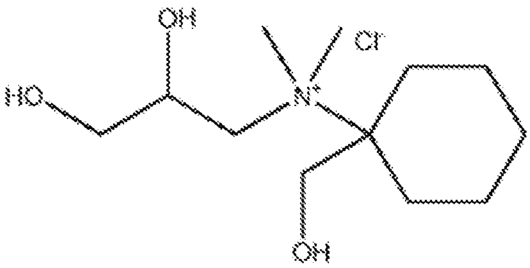

2,3 dihydroxypropyldimethylcyclohexanemethanolammonium chloride

In some cases, quaternary salts like those shown above are obtained as a mixture of quaternary salts, including byproducts which usually are structurally related and which may have similar properties. In this event, the amount of the quaternary salt in the composition is the total weight of the salt as obtained including byproducts, but excluding any water.

Preferably, the aliphatic quaternary ammonium salt is present in the composition in an amount from 0.5 wt% to 6 wt%, preferably at least 0.8 wt%, preferably at least 1 wt%, preferably from at least 1.2 wt%, preferably at least 1.4 wt%, preferably at least 1.6 wt%, preferably at least 1.8 wt%; preferably no more than 5 wt%, preferably no more than 4 wt%, preferably no more than 3.5 wt%, preferably no more than 3 wt%. Percentages are on a solids basis, i.e., the salt itself, exclusive of any water it contains.

The surfactant package is one or more surfactants added to the composition. Preferably, the surfactant package comprises more than one surfactant. Preferably, the surfactant package has a mixture HLB value from 5 to 10, preferably at least 5.5, preferably at least 6, preferably at least 6.5, preferably at least 6.7; preferably no greater than 9, preferably no greater than 8, preferably no greater than 7.5, preferably no greater than 7.3. Mixture HLB value is calculated as the weighted average of the individual HLB values of the surfactants based on their weight percentages in the surfactant package. Preferably, the surfactant package comprises a first surfactant (or combination of surfactants) having an HLB value from 3 to 6, preferably at least 3.5, preferably at least 3.8; preferably no greater than 5.5, preferably no greater than 5, preferably no greater than 4.8. Preferably, the first surfactant is present in the surfactant package in an amount from 50 to 85 wt%, preferably from 55 to 76 wt%, preferably from 62 to 74 wt%. Preferably, the first surfactant is a

monoester of a fatty acid and a tetrahydrofuran compound having 3-4 hydroxyl groups and 5-6 carbon atoms, preferably sorbitan. Preferably, the fatty acid has at least sixteen carbon atoms, preferably eighteen carbon atoms. Preferably, the first surfactant is combined with one or more additional surfactants having higher HLB values to achieve the desired average. Preferably, the surfactant package comprises a second surfactant (or combination of surfactants) having an HLB value from 14 to 19, preferably from 15 to 18. Preferably, the second surfactant is present in the surfactant package in an amount from 10 to 25 wt%, preferably from 12 to 20 wt%, preferably from 14 to 19 wt%. Preferably, the second surfactant is monosaccharide ester of a fatty acid in which the monosaccharide also is substituted with from 12 to 30 polymerized ethylene oxide units (preferably from 15 to 25); preferably the fatty acid has from eight to sixteen carbon atoms, preferably from ten to fourteen. Preferably, the surfactant package comprises a third surfactant (or combination of surfactants) having an HLB value from 5 to 12, preferably from 6 to 11, preferably from 7 to 10. Preferably, the third surfactant is present in the surfactant package in an amount from 10 to 25 wt%, preferably from 12 to 20 wt%, preferably from 13 to 17 wt%. Preferably, the third surfactant is monosaccharide ester of a fatty acid, preferably a fatty acid having from eight to sixteen carbon atoms, preferably from ten to fourteen.

Preferably, the surfactant package is present in the composition in an amount from 15 to 30 wt%, preferably at least 17 wt%, preferably at least 19 wt%, preferably at least 20 wt%, preferably at least 21 wt%; preferably no greater than 28 wt%, preferably no greater than 27 wt%, preferably no greater than 26 wt%, preferably no greater than 25.5 wt%.

Preferably, the composition comprises water in an amount from 1 to 10 wt%, preferably at least 2 wt%, preferably at least 2.5 wt%, preferably at least 3 wt%, preferably at least 3.5 wt%; preferably no greater than 8 wt%, preferably no greater than 7 wt%, preferably no greater than 6 wt%, preferably no greater than 5 wt%.

Preferably, the ingredients of the composition are blended in the form of a microemulsion, preferably a water-in-oil (w/o) microemulsion, preferably at a temperature from 15°C to 35°C. Microemulsions are isotropic, thermodynamically stable transparent (or translucent) systems of oil, water and surfactant, frequently in combination with a co-surfactant and with a droplet size usually in the range of 10 to 200 nm, preferably from 20 to 100 nm, preferably from 30 to 60 nm. They are formed spontaneously or with a little input of energy and therefore are simple to prepare. Formation of microemulsions is not process dependent, i.e., the order of addition of starting materials or speed/type of mixing is not critical to the preparation of the microemulsions, which is highly advantageous for large-scale production.

Preferably, the composition is substantially free of silicones, i.e., it has no more than 5 wt% silicones, preferably no more than 2 wt%, preferably no more than 1 wt%, preferably no more than 0.5 wt%, preferably no more than 0.2 wt%, preferably no more than 0.1 wt%, preferably no more than 0.05 wt%.

Preferably, the composition has three main phases :-

a. Aqueous phase consisting of water + aliphatic quaternary ammonium salt
b. Oil phase consisting of dehydrated vegetable oil
c. The surfactant package

EXAMPLES

The surfactant system consists of polyoxyethylene sorbitan monolaurate (TWEEN20), sorbitan monolaurate (SPAN20) and sorbitan monooleate (SPAN 80). TWEEN 20 is hydrophilic whereas SPAN 20 and SPAN 80 are hydrophobic. The ternary surfactant system was based on the proportion shown in the table below and has a mixture HLB value of 7.

| | HLB | wt% |
|---|---|---|
| **Tween 20** | 16.7 | 16.6 |
| **Span 20** | 8.6 | 15 |
| **Span 80** | 4.3 | 68.4 |
| **Mixture** | 7 | 100 |

Virgin Coconut Oil (VCO) is filtered using filter paper in the presence of anhydrous sodium sulfate to obtain dehydrated VCO and was used as the continuous phase.

In theory, the micro-emulsion system has three main phases:

a. Aqueous phase consisting of Water + hydrophilic active, which in this case is a 50% Glycerol Quat solution
b. Oil phase consisting of dehydrated VCO
c. The ternary surfactant system

The surfactant to aqueous phase ratio (weight basis) is fixed at 4.5:1 and the VCO to Aqueous phase + Surfactant ratio (weight basis) is fixed at 2.3:1.

Shown in the Table below is a representative formulation for preparing a 1% active Glycerol Quat based micro-emulsion.

| **Constituent** | **Wt in gms** |
|---|---|
| Glycerol Quat (50% aq. solution) | 2 |
| DI water | 3.2 |
| TWEEN-20 | 3.9 |
| SPAN -20 | 3.5 |
| SPAN -80 | 16 |
| dehydrated VCO | 71.4 |
| Total | 100 |

The final product (micro-emulsion) is prepared by following the procedure below:

1. All the ingredients were measured to required quantities.
2. The ternary surfactant system is mixed with DI water and Glycerol Quat (50% solution) on a hot plate stirrer
3. Stir at a medium speed for 10 minutes while maintain the temperature to 38-40 °C
4. The resulting mixture was cooled to around 30-31 °C while continuing to stir
5. Dehydrated VCO is added dropwise while stirring at high speed and maintaining the temperature to ~ 30°C.
6. The presence of transparent micro-emulsion mixture indicates the optimum ratio of water/surfactant/oil.

Coconut oil based water-in-oil (w/o) micro-emulsions were prepared with a Glycerol Quat loading of 2.75% and 5% - using the procedure described above. The samples were then compared for conditioning improvement against virgin coconut hair oil (VCO) and a blank micro-emulsion (i.e. a water-in-oil microemulsion with no Glycerol Quat).

**Wet combing**

The test is performed on a DIASTRON MTT 175 Hair Combing Machine. This machine uses a tensile tester to measure the force required to pull a comb through a hair tress before and after treatment with a product.
Virgin hair tresses were obtained from International Hair importers and Products Inc. (NY, USA). The following procedure was performed to measure wet combing resistance on DAISTRON MTT 175 Hair Combing machine:

1. The hair tress was washed and dried using the standard procedure
2. The hair tress measuring 200mm in length was applied with 0.5gm of the product and was gently massaged for 1 minute.
3. The treated tress is then clamped to the load cell of the MTT 175 and a hard rubber comb is attached to the moving bridge of the tensile tester.
4. The tress is combed at the rate of 500 mm/min speed setting available in the machine.
5. The resistance to combing is measured as the variation of gm-force experienced by the hair tress with respect to the length of the tress.

Table 1. Wet Combing Resistance

| VCO | | Blank | | 2.75% Glycerol Quat | | 5% Glycerol Quat | |
|---|---|---|---|---|---|---|---|
| Length of hair | Load (gm-force) | Length of hair | Load (gm-force) | Length of hair | Load (gm-force) | Length of hair | Load (gm-force) |
| 21.6 | 6.5 | 20.0 | 8.6 | 20.3 | 8.0 | 21.0 | 3.7 |
| 26.1 | 8.4 | 21.9 | 10.0 | 21.9 | 7.8 | 23.2 | 4.6 |
| 29.3 | 10.1 | 25.5 | 11.0 | 22.9 | 8.3 | 25.2 | 5.5 |

(continued)

| VCO | | Blank | | 2.75% Glycerol Quat | | 5% Glycerol Quat | |
|---|---|---|---|---|---|---|---|
| Length of hair | Load (gm-force) | Length of hair | Load (gm-force) | Length of hair | Load (gm-force) | Length of hair | Load (gm-force) |
| 32.2 | 11.6 | 29.6 | 12.2 | 25.1 | 7.9 | 26.8 | 5.7 |
| 36.4 | 13.5 | 33.5 | 11.7 | 28.6 | 8.0 | 30.3 | 5.8 |
| 40.9 | 14.3 | 37.0 | 12.2 | 30.2 | 8.9 | 32.2 | 6.5 |
| 46.7 | 16.4 | 43.4 | 11.3 | 35.0 | 9.0 | 35.8 | 6.6 |
| 54.8 | 21.1 | 50.7 | 9.3 | 40.4 | 9.0 | 39.3 | 6.2 |
| 62.5 | 21.6 | 66.4 | 9.9 | 42.0 | 9.2 | 44.2 | 6.4 |
| 68.0 | 21.9 | 76.0 | 9.4 | 47.1 | 9.5 | 47.1 | 6.0 |
| 72.8 | 21.1 | 85.0 | 9.6 | 52.9 | 9.7 | 49.0 | 5.7 |
| 77.7 | 19.4 | 90.7 | 9.6 | 56.4 | 10.2 | 53.2 | 6.2 |
| 83.8 | 18.9 | 95.5 | 9.6 | 69.7 | 8.8 | 59.3 | 6.2 |
| 88.6 | 17.9 | 101.3 | 10.9 | 78.4 | 7.5 | 65.1 | 5.5 |
| 91.8 | 18.9 | 107.4 | 10.9 | 84.1 | 7.3 | 69.3 | 5.8 |
| 99.3 | 17.9 | 111.5 | 11.1 | 87.6 | 6.8 | 71.2 | 5.1 |
| 103.8 | 16.9 | 115.7 | 11.0 | 94.3 | 7.7 | 75.1 | 4.7 |
| 109.2 | 16.7 | 121.1 | 10.7 | 98.8 | 7.4 | 80.9 | 4.4 |
| 114.1 | 16.5 | 127.9 | 11.3 | 106.1 | 7.8 | 86.0 | 4.3 |
| 118.6 | 16.1 | 134.3 | 11.4 | 115.3 | 8.3 | 93.1 | 4.3 |
| 123.7 | 16.0 | 138.4 | 12.2 | 123.0 | 8.7 | 99.9 | 4.2 |
| 127.9 | 17.2 | 142.3 | 12.1 | 129.7 | 9.8 | 105.7 | 4.5 |
| 131.8 | 17.2 | 150.3 | 12.2 | 136.1 | 9.0 | 113.8 | 4.1 |
| 137.9 | 16.1 | 156.7 | 12.6 | 143.7 | 8.5 | 121.5 | 3.7 |
| 143.1 | 16.9 | 160.2 | 12.7 | 152.6 | 7.8 | 127.9 | 3.6 |
| 147.6 | 17.2 | 162.1 | 12.9 | 154.5 | 7.1 | 134.7 | 3.3 |
| 156.0 | 18.6 | 166.6 | 15.2 | 160.0 | 7.7 | 138.6 | 3.8 |
| 161.8 | 19.1 | 169.5 | 14.9 | 164.7 | 6.9 | 143.7 | 3.8 |
| 164.7 | 17.8 | 172.3 | 14.9 | 168.9 | 7.1 | 150.2 | 3.7 |
| 167.9 | 18.2 | 175.2 | 14.2 | 173.7 | 6.2 | 157.9 | 4.3 |
| 174.0 | 22.8 | 177.8 | 13.9 | 178.8 | 5.9 | 163.4 | 4.8 |
| 177.5 | 23.8 | 180.0 | 13.9 | 181.6 | 6.1 | 169.2 | 5.3 |
| 186.2 | 16.4 | 183.9 | 14.1 | 185.8 | 6.1 | 175.0 | 5.1 |
| 189.1 | 16.8 | 186.1 | 14.1 | 188.3 | 5.8 | 182.0 | 5.3 |
| 193.6 | 16.7 | 193.5 | 12.2 | 192.5 | 6.1 | 188.5 | 4.8 |
| 197.8 | 16.0 | 196.3 | 11.8 | 196.3 | 5.8 | 195.6 | 4.9 |
| 200.4 | 14.9 | 199.9 | 10.4 | 200.8 | 5.6 | 198.5 | 4.5 |

As shown in Table 1, the water-in-oil microemulsion containing 5% Glycerol Quat provides the least combing resistance amongst all samples tested and reduces the peak combing force by about 70% as compared to control (Virgin

Coconut Oil). The blank microemulsion also provides a conditioning effect and as expected lowers combing resistance significantly (about 58% as compared to control).

**Dry Combing**

The hair samples from wet combing study were washed for 1 minute under flowing tap water without any soap or shampoo. The resulting samples were completely oil-free and without any tackiness or grease - whereas on the other hand the control sample still had oil on the hair fibers and was greasy to touch. The control swatch was then washed with a commercial 2-in-1 shampoo (containing conditioner) to remove the oil. All the test samples were then dried for 4 hours before conducting the dry combing test.

The dry combing test was performed on the same set of four hair samples obtained from wet combing:

blank microemulsion,
microemulsion with 2.75% Glycerol Quat (50%),
microemulsion with 5% Glycerol Quat (50%)
virgin coconut hair oil applied swatch washed with a 2-in-1 shampoo

Table 2. Dry Combing Resistance

| Samples | Peak Load (gm-force) |
|---|---|
| Blank Microemulsion | 75.2 |
| w/o microemulsion - 2.75% Glycerol Quat | 47.2 |
| w/o microemulsion - 5% Glycerol Quat | 15.9 |
| Virgin Coconut Oil swatch washed with 2-in-1 shampoo | 12.1 |

The blank microemulsion does not provide any conditioning on dry hair mainly because of the absence of the conditioning active i.e. Glycerol Quat. The hair samples applied with Glycerol Quat containing microemulsion provide improved conditioning with increasing concentration mainly due to the deposition of the cationic polyhydric salt on the hair shaft as demonstrated by the drop in peak load in Table 2. The treatment of a hair sample with 5% Glycerol Quat microemulsion formulation achieves almost equivalent dry conditioning imparted on the control (Virgin coconut Oil) hair sample after washing with a commercial silicone containing 2-in-1 shampoo.

**Claims**

1. A composition comprising: (a) from 60 to 85 wt% vegetable oil; (b) an aliphatic quaternary ammonium salt having from five to twenty carbon atoms and from two to six hydroxyl groups; and (c) a surfactant package.

2. The composition of claim 1 in which the surfactant package has a weight average HLB value from 5 to 10.

3. The composition of claim 2 in which the composition comprises from 65 to 77 wt% vegetable oil.

4. The composition of claim 3 in which the aliphatic quaternary ammonium salt has from five to sixteen carbon atoms and from two to four hydroxyl groups and in which the aliphatic quaternary ammonium salt is a saturated aliphatic compound having only one nitrogen atom.

5. The composition of claim 4 in which the composition comprises from 0.5 to 6 wt% of the aliphatic quaternary ammonium salt.

6. The composition of claim 4 in which the composition comprises from 15 to 30 wt% of the surfactant package.

7. A method for preparing a conditioned hair oil; said method comprising combining with a hair oil: (a) an aliphatic quaternary ammonium salt having from five to twenty carbon atoms and from two to six hydroxyl groups; and (b) a surfactant package.

8. The method of claim 7 in which the surfactant package has a weight average HLB value from 5 to 10.

9. The method of claim 8 in which the aliphatic quaternary ammonium salt has from five to sixteen carbon atoms and from two to four hydroxyl groups and in which the aliphatic quaternary ammonium salt is a saturated aliphatic compound having only one nitrogen atom.

10. The method of claim 9 in which the composition comprises from 65 to 77 wt% vegetable oil, from 0.5 to 6 wt% of the aliphatic quaternary ammonium salt. and from 15 to 30 wt% of the surfactant package.

**Patentansprüche**

1. Eine Zusammensetzung, die Folgendes beinhaltet: (a) zu 60 bis 85 Gew.-% Pflanzenöl; (b) ein aliphatisches quartäres Ammoniumsalz mit fünf bis zwanzig Kohlenstoffatomen und zwei bis sechs Hydroxylgruppen und (c) eine Tensidpackung.

2. Zusammensetzung gemäß Anspruch 1, wobei die Tensidpackung einen HLB-Wert im Gewichtsmittel von 5 bis 10 aufweist.

3. Zusammensetzung gemäß Anspruch 2, wobei die Zusammensetzung zu 65 bis 77 Gew.-% Pflanzenöl beinhaltet.

4. Zusammensetzung gemäß Anspruch 3, wobei das aliphatische quartäre Ammoniumsalz fünf bis sechzehn Kohlenstoffatome und zwei bis vier Hydroxylgruppen aufweist und wobei das aliphatische quartäre Ammoniumsalz eine gesättigte aliphatische Verbindung mit nur einem Stickstoffatom ist.

5. Zusammensetzung gemäß Anspruch 4, wobei die Zusammensetzung zu 0,5 bis 6 Gew.-% das aliphatische quartäre Ammoniumsalz beinhaltet.

6. Zusammensetzung gemäß Anspruch 4, wobei die Zusammensetzung zu 15 bis 30 Gew.-% die Tensidpackung beinhaltet.

7. Ein Verfahren zum Zubereiten eines konditionierten Haaröls; wobei das Verfahren das Vereinigen von Folgendem mit einem Haaröl beinhaltet: (a) einem aliphatischen quartären Ammoniumsalz mit fünf bis zwanzig Kohlenstoffatomen und zwei bis sechs Hydroxylgruppen; und (b) einer Tensidpackung.

8. Verfahren gemäß Anspruch 7, wobei die Tensidpackung einen HLB-Wert im Gewichtsmittel von 5 bis 10 aufweist.

9. Verfahren gemäß Anspruch 8, wobei das aliphatische quartäre Ammoniumsalz fünf bis sechzehn Kohlenstoffatome und zwei bis vier Hydroxylgruppen aufweist und wobei das aliphatische quartäre Ammoniumsalz eine gesättigte aliphatische Verbindung mit nur einem Stickstoffatom ist.

10. Verfahren gemäß Anspruch 9, wobei die Zusammensetzung zu 65 bis 77 Gew.-% Pflanzenöl, zu 0,5 bis 6 Gew.-% das aliphatische quartäre Ammoniumsalz und zu 15 bis 30 Gew.-% die Tensidpackung beinhaltet.

**Revendications**

1. Une composition comprenant : (a) de 60 à 85 % en poids d'huile végétale ; (b) un sel d'ammonium quaternaire aliphatique ayant de cinq à vingt atomes de carbone et de deux à six groupes hydroxyle ; et (c) un ensemble tensioactif.

2. La composition de la revendication 1 dans laquelle l'ensemble tensioactif a une valeur HLB moyenne en poids de 5 à 10.

3. La composition de la revendication 2, la composition comprenant de 65 à 77 % en poids d'huile végétale.

4. La composition de la revendication 3 dans laquelle le sel d'ammonium quaternaire aliphatique a de cinq à seize atomes de carbone et de deux à quatre groupes hydroxyle et dans laquelle le sel d'ammonium quaternaire aliphatique

est un composé aliphatique saturé ayant seulement un atome d’azote.

**5.** La composition de la revendication 4, la composition comprenant de 0,5 à 6 % en poids du sel d’ammonium quaternaire aliphatique.

**6.** La composition de la revendication 4, la composition comprenant de 15 à 30 % en poids de l’ensemble tensioactif.

**7.** Une méthode pour préparer une huile capillaire conditionnée ; ladite méthode comprenant la combinaison avec une huile capillaire : (a) d’un sel d’ammonium quaternaire aliphatique ayant de cinq à vingt atomes de carbone et de deux à six groupes hydroxyle ; et (b) d’un ensemble tensioactif.

**8.** La méthode de la revendication 7 dans laquelle l’ensemble tensioactif a une valeur HLB moyenne en poids de 5 à 10.

**9.** La méthode de la revendication 8 dans laquelle le sel d’ammonium quaternaire aliphatique a de cinq à seize atomes de carbone et de deux à quatre groupes hydroxyle et dans laquelle le sel d’ammonium quaternaire aliphatique est un composé aliphatique saturé ayant seulement un atome d’azote.

**10.** La méthode de la revendication 9 dans laquelle la composition comprend de 65 à 77 % en poids d’huile végétale, de 0,5 à 6 % en poids du sel d’ammonium quaternaire aliphatique, et de 15 à 30 % en poids de l’ensemble tensioactif.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7282471 B
- WO 2012050769 A